Europäisches Patentamt

(19) European Patent Office

Office européen des brevets

(11) Veröffentlichungsnummer: **0 196 271**
**B1**

(12) # EUROPÄISCHE PATENTSCHRIFT

(45) Veröffentlichungstag der Patentschrift:
08.02.89

(51) Int. Cl.⁴: **C 07 J 1/00,** A 61 K 31/565

(21) Anmeldenummer: **86730052.7**

(22) Anmeldetag: **20.03.86**

(54) **Glykoester des Estradiols und Estriols.**

(30) Priorität: **27.03.85 DE 3511587**

(43) Veröffentlichungstag der Anmeldung:
**01.10.86 Patentblatt 86/40**

(45) Bekanntmachung des Hinweises auf die Patenterteilung:
**08.02.89 Patentblatt 89/6**

(84) Benannte Vertragsstaaten:
**AT BE CH DE FR GB IT LI LU NL SE**

(56) Entgegenhaltungen:
**EP-A- 0 003 794**
**FR-A- 1 295 360**
**GB-A- 2 028 336**

(73) Patentinhaber: **SCHERING AKTIENGESELLSCHAFT
Berlin und Bergkamen,
Müllerstrasse 170/178 Postfach 65 03 11,
D-1000 Berlin 65 (DE)**

(72) Erfinder: **Düsterberg, Bernd, Dr., Spirdingseestrasse 27,
D-1000 Berlin 49 (DE)**
Erfinder: **Acksteiner, Bernard, Dr.,
Ludwigkirchstrasse 9 a, D-1000 Berlin 15 (DE)**
Erfinder: **Schulze, Paul-Eberhard, Dr., Endestrasse 30,
D-1000 Berlin 39 (DE)**

**Beschreibung**

Die Erfindung betrifft Glykoester des Estradiols (E₂) und Estriols (E₃) der allgemeinen Formel I

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe dargestellt, sowie Verfahren zu ihrer Herstellung und wässrige Kristallsuspensionen dieser Ester.

Die natürlichen östrogenen Hormone Estradiol und Estriol haben bei der medizinischen Anwendung den Nachteil, dass sie schlecht löslich sind und rasch verstoffwechselt werden.

Durch Veresterung der natürlichen Östrogene mit längerkettigen Carbonsäuren erhält man östrogene Wirkstoffe, die als ölige Lösungen appliziert werden können und eine Langzeitwirkung aufweisen.

In Form öliger Lösungen werden zum Beispiel angewendet Estradiol-17-valerianat, 17-önanthal, 17-undecylat, 17-cypionat und 3-benzoat; ferner wird ein in Wasser löslicher bernsteinsaurer Ester von Estriol, das Estriol-16, 17-di-hemisuccinatnatrium, medizinisch angewendet.

In GB-A-2 028 336 werden durch Halogen oder eine Amino-, Hydroxy- oder Carboxygruppe substituierte Steroidester beschrieben, die mit einer reaktiven Gruppe eines Antitumormittels umgesetzt werden.

Wässrige Kristallsuspensionen von Estradiol- und Estriolestern sind bisher nicht bekannt.

Es wurde nun gefunden, dass die neuen Glykoester des Estradiols und des Estriols der allgemeinen Formel I kristallisierbare Verbindungen darstellen, die sich zur Herstellung von Mikrokristallsuspensionen eignen.

Es wurde ferner gefunden, dass zum Beispiel eine Kristallsuspension von 0,5 mg Östradiol-17-glykobenzoat mit einer prozentualen Verteilung der Kristallgrössen von

15 Gewichtsprozent der Grösse 3 - 10 µm,

60 Gewichtsprozent der Grösse 10 - 26 µm und

25 Gewichtsprozent der Grösse 26 - 40 µm nach einmaliger intramuskulärer Injektion beim Pavian einen 4 Wochen anhaltenden, ausreichend hohen und weitgehend gleichmässig verlaufenden Estradiolspiegel im Plasma liefert.

Mit den bekannten Estradiolestern, die eine geringere Lipophilität aufweisen, lassen sich so gleichmässige und anhaltende Estradiolspiegel nicht erzielen.

Die galenische Formulierung der neuen Ester als Kristallsuspension bietet weiterhin den Vorteil, dass sie mit entsprechenden Estern des Norethisterons oder der von Norethisteron abgeleiteten Steroide, wie zum Beispiel Levonorgestrel, Gestoden, Desogestrel und Lynestrenol, in einer wässrigen Kristallsuspension als Kontrazeptiva eingesetzt werden können. Beispielsweise können 10 - 50 mg Steroidglykoester vom Norethisterontyp und 0,5 - 10 mg Steroidglykoester vom Estradiol- oder Estrioltyp in Form einer wässrigen Kristallsuspension zu einer 1-Monatsspritze für die Kontrazeption bereitgestellt werden.

Nach bekannten Methoden wird eine Mikrokristallsuspension eines Glykoesters des Estradiols oder Estriols der allgemeinen Formel I hergestellt und so gesiebt, dass man 3 Fraktionen folgender Teilchengrösse erhält:

a) 3 - 10 µm,
b) 10 - 30 µm und
c) 25 - 50 µm.

0 - 30 Gewichtsprozent a)
40 - 90 Gewichtsprozent b) und
10 - 30 Gewichtsprozent c)

werden etwa 5 Minuten im Wirbelstrom gemischt, mit physiologischer Kochsalzlösung, gegebenenfalls unter Zusatz eines Stabilisators wie Polyethylenstearat (Myrj(R)), aufgefüllt und hitzesterilisiert, oder aseptisch abgefüllt.

Die Erfindung betrifft auch ein Verfahren zur Herstellung von Glykoestern des Estradiols (E₂) und Estriols (E₃) der allgemeinen Formel I

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe darstellt, dadurch gekennzeichnet, dass man Estradiol oder Estriol mit der Säure Z-CH₂-COOH oder einem Derivat der Säure verestert und gegebenenfalls den Estradiol-diester oder den Estriol-triester partiell in 3-Stellung verseift.

*Beispiel 1*

In einer aseptischen und pyrogenfreien Schallfixapparatur werden 0,5 g Estradiol-17β-benzoyloxyacetat in 5 ml Myrj(R)-Kochsalzlösung (0,9 gewichtsprozentige Kochsalzlösung mit 0,085 Gewichtsprozent Myrj(R)) 3 Minuten beschallt und die erhaltene Suspension wird fraktioniert gesiebt. Man erhält eine Teilchengrössenverteilung von

a) 3 - 10 µm,
b) 10 - 26 µm und
c) 26 - 40 µm.

Die Fraktionen a), b) und c) werden im Verhältnis 15:60:25 im Wirbelstrom gemischt und mit Myrj(R)-Kochsalzlösung auf 100 ml aufgefüllt.

*Beispiel 2*

Estradiol-3,17β-di-acetoxyacetat

Zu einer Lösung von 1,24 g (4,5 mMol) Estradiol in 7,5 ml Collidin und 7,5 ml Tetrachlorethylen werden unter Stickstoff bei 120 °C innerhalb von 2 Stunden 3 g (21,9 mMol) Acetoxyacetylchlorid in 10 ml Tetrachlorethylen unter Rühren zugetropft. Die abgekühlte Lösung gibt man zu 5 g Oxalsäure in 20 ml Wasser und rührt noch 20 Minuten. Es wird mit Methylenchlorid verdünnt und die organische Phase mit Natriumhydrogencarbonatlösung und Wasser ausgeschüttelt und über Natriumsulfat getrocknet. Der Rückstand wird der Niederdruckchromatographie an Kieselgel, Laufmittel Methylenchlorid/Methylenchlorid-Aceton (1 - 2 %) unterworfen. Man erhält 1,23 g Estradiol-3,17β-di-acetoxyacetat vom Schmelzpunkt 95 °C.

*Beispiel 3*

Estradiol-17β-acetoxyacetat

0,9 g Estradiol-3,17β-di-acetoxyacetat werden in 10 ml Aceton gelöst, 5 ml Kaliumcarbonatlösung (250 mg Kaliumcarbonat in 5 ml Wasser und 45 ml Methanol) zugegeben und 10 Minuten gerührt. Dann säuert man an und destilliert Methanol und Aceton im Vakuum ab. Der Rückstand wird in 10 ml Methylenchlorid aufgenommen, mit Natriumhydrogencarbonatlösung und Wasser gewaschen, über Natriumsulfat getrocknet und eingedampft. Der Rückstand wird der Niederdruckchromatographie an Kieselgel, Laufmittel Methylenchlorid/Methylenchlorid-Aceton (0,5 - 1 %) unterworfen. Man erhält 0,28 g Estradiol-17β-acetoxyacetat vom Schmelzpunkt 170 - 172 °C.

Als Nebenprodukt der Verseifung mit Kaliumcarbonat gemäss Beispiel 4 erhält man Estradiol-17β-glykolat vom Schmelzpunkt 231 - 236 °C.

*Beispiel 4*

Estradiol-3,17β-di-benzoyloxyacetat

Zu einer Lösung von 1,36 g (5 mMol) Estradiol in 7,5 ml Collidin und 7,5 ml Tetrachlorethylen tropft man unter Stickstoff bei 120 °C Badtemperatur innerhalb von 2 Stunden 2 g (10 mMol) Benzoyloxyacetylchlorid. Danach wird die abgekühlte Lösung zu 5 g Oxalsäure in 20 ml Wasser gegossen und 20 Minuten gerührt. Die organische Phase schüttelt man mit Natriumhydrogencarbonatlösung und Wasser aus und trocknet über Natriumsulfat. Der Rückstand wird der Niederdruckchromatographie an Kieselgel, Laufmittel Methylenchlorid unterworfen. Man erhält 1,79 g Estradiol-3,17β-di-benzoyloxyacetat vom Schmelzpunkt 105 °C.

Benzoylacetoxyacetylchlorid erhält man durch Umsetzung von Benzoylacetoxyessigsäure mit Oxalylchlorid.

*Beispiel 5*

Estradiol-17β-benzoyloxyacetat

1,79 g Estradiol-3,17β-di-benzoyloxyacetat werden in 32 ml Aceton gelöst und nach Zugabe von 10 ml einer Kaliumcarbonatlösung (250 mg Kaliumcarbonat in 5 ml Wasser und 45 ml Methanol) 10 Minuten lang gerührt. Danach säuert man an und destilliert Methanol und Aceton im Vakuum ab. Der organische Rückstand wird in 10 ml Methylenchlorid aufgenommen, mit Natriumhydrogencarbonat und Wasser gewaschen und über Natriumsulfat getrocknet. Der Rückstand wird der Niederdruckchromatographie an Kieselgel, Laufmittel Methylenchlorid/Methylenchlordi-Aceton bis 1 % unterworfen. Man erhält 0,79 g Estradiol-17β-benzoyloxyacetat vom Schmelzpunkt 153 - 155 °C.

*Beispiel 6*

Estriol-3,16α, 17β-triacetoxyacetat

Zu einer Lösung von 1,45 g (5 mMol) Estriol in 20 ml Tetrachlorethylen und 7,5 ml Collidin tropft man unter Stickstoff bei 120 °C Badtemperatur innerhalb von 2 Stunden 5 g (37 mMol) Acetoxyacetylchlorid. Danach wird die abgekühlte Lösung zu 5 g Oxalsäure in 20 ml Wasser gegossen und 20 Minuten gerührt. Die organische Phase schüttelt man mit Natriumhydrogencarbonatlösung und Wasser aus und trocknet über Natriumsulfat. Der Rückstand wird der Niederdruckchromatographie an Kieselgel, Laufmittel Methylenchlorid unterworfen. Man erhält 1,15 g Estriol-3,16α, 17β-triacetoxyacetat vom Schmelzpunkt 70 °C.

*Beispiel 7*

Estriol-16α, 17β-diacetoxyacetat

1,15 g Estriol-3,16α, 17β-triacetoxyacetat werden in 5 ml Aceton gelöst, 5 ml Kaliumcarbonatlösung (250 mg Kaliumcarbonat in 5 ml Wasser und 45 ml Methanol) zugegeben und 10 Minuten gerührt. Danach säuert man an und destiliert das Lösungsmittel ab. Der Rückstand wird in 10 ml Methylenchlorid aufgenommen, mit Natriumhydrogencarbonatlösung und Wasser gewaschen und über Natriumsulfat getrocknet und eingedampft. Der Rückstand wird der Niederdruckchromatographie an Kieselgel, Laufmittel Methylenchlorid/Methylenchlorid-Aceton 0,5 - 1 % unterworfen. Man erhält 0,52 g Estriol-16α, 17β-diacetoxyacetat vom Schmelzpunkt 170 °C.

*Beispiel 8*

Estriol-3,16α, 17β-tribenzoyloxyacetat

Zu einer Lösung von 0,95 g (3,3 mMol) Estriol in 7,5 ml Tetrachlorethylen und 7,5 ml Collidin tropft man unter Stickstoff bei 120 °C Badtemperatur innerhalb von 2 Stunden eine Lösung von 2 g (10 mMol) Benzoyloxyacetylchlorid in 10 ml Tetrachlorethylen. Danach wird die abgekühlte Lösung zu 5 g Oxalsäure in 20 ml Wasser gegossen und 20 Minuten gerührt. Die organische Phase schüttelt man mit Natriumhydrogencarbonatlösung und Wasser aus und trocknet über Natriumsulfat. Der Rückstand wird der Niederdruckchromatographie an Kieselgel, Laufmittel Methylenchlorid/Methylenchlorid-Aceton (0,5 - 2 %) unterworfen. Man erhält 1,36 g Estriol 3,16α, 17β-tribenzoyloxyacetat vom Schmelzpunkt 95 °C.

*Beispiel 9*

Estriol-16α, 17β-dibenzoyloxyacetat

1,36 g Estriol-3,16α, 17β-tribenzoyloxyacetat werden in 7 ml Aceton gelöst, 6 ml Kaliumcarbonatlösung (125 mg Kaliumcarbonat in 5 ml Wasser und 45 ml Methanol) zugegeben und 10 Minuten gerührt. Danach säuert man an und destilliert das Lösungsmittel ab. Der Rückstand wird in 10 ml Methylenchlorid aufgenommen, mit Natriumhydrogencarbonatlösung und Wasser gewaschen und über Natriumsulfat getrocknet und eingedampft. Der Rückstand wird der Niederdruckchromatographie an Kieselgel, Laufmittel Methylenchlorid/Methylenchlorid-Aceton bis 1 % unterworfen. Man erhält 0,56 g Estriol-16α, 17β-dibenzoyloxyacetat vom Schmelzpunkt 160 °C.

**Patentansprüche für die Vertragsstaaten:**
BE, CH, LI, DE, FR, GB, IT, LU, NL, SE

1. Glykoester des Estradiols (E₂) und Estriols (E₃) der allgemeinen Formel I

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe darstellt.

2. Monoglykoester des Estradiols in 17-Stellung.
Estradiol-17β-acetoxyacetat.
Estradiol-17β-benzoyloxyacetat.
Estradiol-17β-glykolat.

3. Diglykoester des Estradiols.
Estradiol-3,17β-di-acetoxyacetat.
Estradiol-3,17β-di-benzoyloxyacetat.

4. Diglykoester des Estriols in 16,17-Stellung.
Estriol-16α, 17β-di-acetoxyacetat.
Estriol-16α, 17β-di-benzoyloxyacetat.

5. Triglykoester des Estriols.
Estriol-3,16α, 17β-tri-acetoxyacetat.
Estriol-3,16α, 17β-tri-benzoyloxyacetat.

6. Wässrige Kristallsuspension von Glykoestern des Estradiols ($E_2$) und Estriols ($E_3$), dadurch gekennzeichnet, dass sie eine Verbindung der allgemeinen Formel I

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe darstellt, in folgenden Fraktionen enthält:
    0 - 30 Gewichtsprozent der Teilchengrösse
        3 - 10 μm,
    40 - 90 Gewichtsprozent der Teilchengrösse
        10 - 30 μm und
    10 - 30 Gewichtsprozent der Teilchengrösse
        25 - 50 μm.

7. Verfahren zur Herstellung von Glykoestern des Estradiols ($E_2$) und Estriols ($E_3$) der allgemeinen Formel 1

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe darstellt, dadurch gekennzeichnet, dass man Estradiol oder Estriol mit der Säure Z-CH₂-COOH oder einem Derivat der Säure verestert und gegebenenfalls den Estradiol-diester oder den Estrioltriester partiell in 3-Stellung verseift.

**Patentanspruch für den Vertragsstaat: AT**

1. Verfahren zur Herstellung von Glykoestern des Estradiols ($E_2$) und Estriols ($E_3$) der allgemeinen Formel I

$$(E_{2,3})\text{-O-CO-CH}_2\text{-Z} \qquad (I),$$

worin

Z eine Hydroxygruppe oder die Gruppe -O-CO-R mit R in der Bedeutung einer Methyl- oder Phenylgruppe darstellt, dadurch gekennzeichnet, dass man Estradiol oder Estriol mit der Säure Z-CH₂-COOH oder einem Derivat der Säure verestert und gegebenenfalls den Estradiol-diester oder den Estrioltriester partiell in 3-Stellung verseift.

**Claims for the Contracting States:**
BE, CH, LI, DE, FR, GB, IT, LU, NL, SE

1. Glycoesters of estradiol ($E_2$) and estriol ($E_3$) of general formula I

$$(E_{2,3})\text{-O-CO-CH}_2\text{-Z} \qquad (I)$$

wherein

Z is a hydroxy group or the group -O-CO-R with R meaning a methyl or phenyl group.

2. Monoglycoesters of estradiol in the 17-position:
estradiol 17β-acetoxyacetate,
estradiol 17β-benzoyloxyacetate,
estradiol 17β-glycolate.

3. Diglycoesters of estradiol:
estradiol 3,17β-diacetoxyacetate,
estradiol 3,17β-dibenzoyloxyacetate.

4. Diglycoesters of estriol in the 16,17-position:
estriol 16α,17β-diacetoxyacetate,
estriol 16α,17β-dibenzoyloxyacetate.

5. Triglycoesters of estriol:
estriol 3,16α, 17β-triacetoxyacetate,
estriol 3,16α,17β-tribenzoyloxyacetate.

6. Aqueous crystalline suspension of glycoesters of estradiol ($E_2$) and estriol ($E_3$), characterised in that it contains a compound of general formula I

$$(E_{2,3})\text{-O-CO-CH}_2\text{-Z} \qquad (I)$$

wherein

Z is a hydroxy group or the group -O-CO-R with R meaning a methyl or phenyl group, in the following fractions:
    0 - 30 % by weight of a particle size of
        3 - 10μm,
    40 - 90 % weight of a particle size of
        10 - 30 μm, and
    10 - 30 % by weight of a particle size of
        25 - 50 μm.

7. A process for the preparation of glycoesters of estradiol ($E_2$) and estriol ($E_3$) of general formula I

$$(E_{2,3})\text{-O-CO-CH}_2\text{-Z} \qquad (I)$$

wherein

Z is a hydroxy group or the group -O-CO-R with R meaning a methyl or phenyl group, characterized in that estradiol or estriol is esterified with the acid Z-CH₂-COOH or a derivative of the acid, and the estradiol diester or the estriol triester is optionally partially hydrolysed in the 3-position.

**Claim for the Contracting State: AT**

1. A process for the preparation of glycoesters of estradiol ($E_2$) and estriol ($E_3$) of general formula I

$$(E_{2,3})\text{-O-CO-CH}_2\text{-Z} \qquad (I)$$

wherein

Z is a hydroxy group or the group -O-CO-R with R meaning a methyl or phenyl group, characterized in that estradiol or estriol is esterified with the acid Z-CH₂-COOH or a derivative of the acid, and the estradiol diester or the estradiol triester is optionally partially hydrolysed in the 3-position.

**Revendications pour les Etats contractants:**
BE, CH, DE, FR, GB, IT, LI, LU, NL, SE

1. Ester glycolique de l'oestradiol ($E_2$) et de l'oestriol ($E_3$), de formule générale I

$(E_{2,3})$-O-CO-$CH_2$-Z            (I),

dans laquelle

Z représente un groupe hydroxyle ou le groupe -O-CO-R, dans lequel R représente un groupe méthyle ou phényle.

2. Monoester glycolique de l'oestradiol en position 17:
Acétoxyacétate-17β d'oestradiol
Benzoyloxyacétate-17β d'oestradiol
Glycolate-17β d'oestradiol.

3. Diester glycolique de l'oestradiol
Di-acétoxyacétate-3,17β d'oestradiol
Di-benzoyloxyacétate-3,17β d'oestradiol.

4. Diester glycolique de l'oestriol, en position 16, 17:
Di-acétoxyacétate-16α-17β d'oestriol.
Di-benzoyloxyacétate-16α-17β d'oestriol.

5. Triester glycolique de l'oestriol
Tri-acétoxyacétate-3,16α-17β d'oestriol
Tri-benzoyloxyacétate-3,16α,17β d'oestriol.

6. Suspension aqueuse de cristaux d'esters glycoliques de l'oestradiol ($E_2$) et de l'oestriol ($E_3$), caractérisée en ce qu'elle contient un composé de formule générale I

$(E_{2,3})$-O-CO-$CH_2$-Z            (I),

dans laquelle

Z représente un groupe hydroxyle ou le groupe -O-CO-R, dans lequel R représente un groupe méthyle ou phényle, selon les fractions suivantes:

0 à 30 % en poids de particules ayant pour grosseur de 3 à 10 μm,
40 à 90 % en poids de particules ayant pour grosseur de 10 à 30 μm, et
10 à 30 % en poids de particules ayant pour grosseur 25 à 50 μm.

7. Procédé pour préparer des esters glycoliques de l'oestradiol ($E_2$) et de l'oestriol ($E_3$), de formule générale I

$(E_{2,3})$-O-CO-$CH_2$-Z            (I),

dans laquelle

Z représente un groupe hydroxyle ou le groupe -O-CO-R, dans lequel R représente un groupe méthyle ou phénile, procédé caractérisé en ce qu'on estérifie l'oestradiol ou l'oestriol avec l'acide Z-$CH_2$-COOH ou avec un dérivé de cet acide et, éventuellement, on saponifie partiellement en position 3 le diester de l'oestradiol ou le triester de l'oestriol.

**Revendication pour l'Etat contractant: AT**

1. Procédé pour préparer des esters glycoliques de l'oestradiol ($E_2$) et de l'oestriol ($E_3$), de formule générale I

$(E_{2,3})$-O-CO-CH.Z            (I),

dans laquelle

Z représente un groupe hydroxyle ou le groupe -O-CO-R, dans lequel R représente un groupe méthyle ou phényle, procédé caractérisé en ce qu'on estérifie l'oestradiol ou l'oestriol avec l'acide Z-$CH_2$-COOH ou avec un dérivé de cet acide et, éventuellement, on saponifie partiellement en position 3 le diester de l'oestradiol ou le triester de l'oestriol.